Europäisches Patentamt

European Patent Office

Office européen des brevets

(19)

(11) EP 0 375 092 B1

(12) EUROPÄISCHE PATENTSCHRIFT

(45) Veröffentlichungstag und Bekanntmachung des Hinweises auf die Patenterteilung:
24.01.1996 Patentblatt 1996/04

(51) Int Cl.6: **C12N 1/21**, C12N 15/70, C12N 15/82, A01H 5/00

(21) Anmeldenummer: 89250117.2

(22) Anmeldetag: 18.12.1989

(54) **Kartoffelknollenspezifische transkriptionale Regulation**

Potato tuber specific transcriptional regulation

Régulation de transcription spécifique pour des tubercules de pomme de terre

(84) Benannte Vertragsstaaten:
AT BE CH DE ES FR GB GR IT LI LU NL SE

(30) Priorität: 21.12.1988 DE 3843627

(43) Veröffentlichungstag der Anmeldung:
27.06.1990 Patentblatt 1990/26

(73) Patentinhaber:
INSTITUT FÜR GENBIOLOGISCHE FORSCHUNG BERLIN GMBH
D-14195 Berlin (DE)

(72) Erfinder:
• Rocha-Sosa, Mario, Dr. Centro de Investigation Cuernavaca, Morelos (MX)
• Sonnewald, Uwe, Dr.
D-1000 Berlin 10 (DE)
• Frommer, Wolf-Bernd, Dr.
D-1000 Berlin 45 (DE)
• Willmitzer, Lothar, Prof. Dr.
D-1000 Berlin 39 (DE)
• Stratmann, Marina
D-1000 Berlin 37 (DE)

(56) Entgegenhaltungen:
• PLANT MOLECULAR BIOLOGY, Band 9, 1987, Seiten 345-375, Martinus Nijhoff Publishers, Dordrecht, NL; D. TWELL et al.: "The 5' flanking DNA of a patatin gene directs tuber specific expression of a chimaeric gene in potato"
• TWO HUNDRED AND EIGHT MEETING OF THE GENETICAL SOCIETY, Norwich, GB, 13.-15. April 1988, Band 62, Nr. 2, Seite 280, Zusammenfassung Nr. 22, HEREDITY; M. BEVAN et al.: "Transcriptional regulation of genes during potato tuberization"
• JOURNAL OF CELLULAR BIOCHEMISTRY, Suppl. 12C, Seite 206 & SYMPOSIUM ON THE MOLECULAR BASIS OF PLANT DEVELOPMENT HELD AT THE 17TH ANNUAL UCLA SYMPOSIA ON MOLECULAR AND CELLULAR BIOLOGY, 26. März -2. April 1988, Zusammenfassung Nr. L. 529; D. TWELL et al.:
• JOURNAL OF CELLULAR BIOCHEMISTRY, Suppl. 12C, Seite 199 & SYMPOSIUM ON THE MOLECULAR BASIS OF PLANT DEVELOPMENT HELD AT HE 17TH ANNUAL UCLA SYMPOSIA 26. März - 2. April 1988, Zusammenfassung Nr. L509; F. HEIDEKAMP et al.: "Expression of foreign genes in solanum tuberosum CVS bintje and desiree"
• JOURNAL OF CELLULAR BIOCHEMISTRY, Supl . 11B, Seite 57 & SYMPOSIUM ON PLANT GENE SYSTEMS AND THEIR BIOLOGY HELD AT 16TH ANNUAL UCLA MEETING ON MOLECULAR AND CELLULAR BIOLOGY, 2.-8. Februar 1987, Zusammenfassung Nr. F434; R.A. JEFFERSON et al.: "Regulated expression of a chimeric patatin-glucuronidase fusion in tubers and induced internode cuttings of transformed potato"
• EMBO JOURNAL, Band 8, Nr. 1, 1989, Seiten 23-29, IRL Press; M. ROCHA-SOSA et al.: "Both developmental and metabolic signals activate the promoter of a class I patatin gene"
• TRENDS BIOTECHNOL. Band 4, 1986, Seiten 314-320; J.M. JAYNES et al.: "Plant protein improvement by genetic engineering: use of synthetic genes"
• GENE, Band 62, 1988, Seiten 27-44, Elsevier Science Publishers B.V. (Biomedical Division); G.A. MIGNERY et al.: "Molecular characterization of the patatin multigene family of potato"

**Beschreibung**

Die vorliegende Erfindung betrifft die Verwendung einer DNA-Sequenz einer Expressions-Kassette, auf der die kartoffelknollenspezifischen regulatorischen Bereiche lokalisiert sind, sowie die Übertragung dieser DNA-Sequenz in das pflanzliche Genom, unter Verwendung von Agrobakterien als Transfermikroorganismen. Die DNA-Sequenz enthält ein Patatin-Gen mit einem Patatin-Gen-Promoter. Die übertragene DNA-Sequenz sorgt sowohl für die Regulierung endogener als auch fur die Herstellung heterologer Produkte in Kulturpflanzen.

Bedingt durch den kontinuierlich steigenden Nahrungsmittel- und Rohstoffbedarf, der aus der ständig wachsenden Weltbevölkerung resultiert, wird es in zunehmendem Maße Aufgabe der biotechnologischen Forschung, sich wegen der langfristig abzusehenden sich verkleinernden Anbauflachen um die Steigerung des Ertrages von Nutzpflanzen und deren Inhaltsstoffe zu bemühen. Eine Steigerung des Ertrages kann u.a. dadurch erreicht werden, daß die Resistenz von Nutzpflanzen gegen Pflanzenschädlinge und Pflanzenkrankheiten und/oder ungünstige Bonverhaltnisse verstärkt wird. Eine Verstärkung der Resistenz könnte z.B. dadurch erreicht werden, daß die Pflanzen induziert und zu einer vermehrten Bildung von Schutzstoffen veranlaßt werden. Hierzu muß der Metabolismus der Pflanze manipuliert werden. Dies kann u.a. durch die Veränderung der im Zellkern enthaltenen Desoxyribonukleinsäure (DNA) hervorgerufen werden. Oft wäre es wünschenswert, in den DNA-Abschnitten, die für die Transkription in einem oder mehreren Teilbereichen der Pflanze oder während eines bestimmten Zeitabschnitts im Pflanzenwachstumszyklus verantwortlich sind, einzugreifen. Daher besteht ein großes Interesse an der Identifizierung der für die Transkription oder Expression endogener Pflanzenprodukte verantwortlichen DNA-Sequenzen im Pflanzengenom. Um derartige DNA-Sequenzen herauszufinden, muß man zunächst nach Produkten suchen, die zu einer bestimmten Zeit im Zellwachstumszyklus oder in einem bestimmten Teil der Pflanze erscheinen. Hat man die dazugehörenden Gene identifiziert und isoliert, ist eine gründliche Untersuchung der Sequenz und vor allem die Identifizierung und Isolierung der gewünschten transkriptionalen regulatorischen Regionen erforderlich. Anschließend müssen geeignete Modelle erstellt werden, deren Funktionen durch Versuche nachgewiesen werden müssen. Die Identifizierung derartiger DNA-Sequenzen ist eine herausfordernde Forschungsaufgabe, die mit beträchtlichen Irrtümern und Ungewißheiten behaftet ist. Es besteht in immer größerem Maße ein Interesse an der Möglichkeit, Pflanzen genetisch zu modifizieren, was die damit verbundenen Aufwendungen und Bemühungen, die mit der Identifizierung transkriptionaler Sequenzen und ihrer Manipulation zu bestimmten Nutzungen verbunden sind, rechtfertigen. Es sind bereits Verfahren zur genetischen Modifikation dikotyler und monokotyler Pflanzen bekannt (EP 267 159), sowie die folgenden Publikationen von Crouch et al., In: Molecular Form and Function of the Plant Genome, eds. van Vloten-Doting, Groot and Hall, Plenum Publishing Corp. 1985, pp 555-566; Crouch and Sussex, Planta (1981) 153:64-74; Crouch et al., J. Mol. Appl. Genet (1983) 2:273-283; und Simon et al., Plant Molecular Biology (1985) 5: 191-201, in denen verschiedene Formen von Speicherproteinen in <u>Brassica</u> <u>napus</u> beschrieben werden und von Beachy et al., EMBO J. (1985) 4:3047-3053; Sengupta-Gopalan et al., Proc. Natl. Acad. Sci. USA (1985) 82:3320-3324; Greenwood and Chrispeels, Plant Physiol. (1985) 79:65-71 and Chen et al., Proc. Natl. Acad. Sci. USA (1986) 83:8560-8564 in denen Untersuchungen, die sich mit Samenspeicherproteinen und genetischer Manipulation befassen, beschrieben werden und von Eckes et al., Mol. Gen. Genet. (1986) 205:14-22 and Fluhr et al., Science (1986) 232:1106-1112, die die genetische Manipulation lichtinduzierter Pflanzengene beschreiben.

Es wird nun eine DNA-Sequenz einer Expressions-Kassette zur Verfügung gestellt, auf der die kartoffelknollenspezifischen regulatorischen Bereiche lokalisiert sind und die ein Patatin-Gen mit einem Patatin-Gen-Promotor enthält, die zur Transformation von Kulturpflanzen verwendet werden kann.

Der DNA-Sequenz, die die regulatorische transkriptionale Starter-Region für die Knollenspezifität enthält, kann eine Sequenz nachgeschaltet werden, die die Informationen für die Modifikation des Phänotyps der betreffenden Zellgewebe und die Bildung sowie mengenmäßige Verteilung endogener Produkte oder die Bildung heterogener Expressionsprodukte für eine neue Funktion enthält. Zweckmässiger Weise sollten die Transkriptions- und Terminations-Regionen in Transkriptions-Richtung durch einen Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker eine Größe von weniger als 100 bp, häufig weniger als 60 bp, mindestens jedoch 5 bp. Der transkriptionale Startbereich kann sowohl nativ bzw. homolog als auch fremdartig bzw. heterolog zur Wirtspflanze sein. Von besonderem Interesse sind die transkriptionalen Startregionen, die mit dem Kartoffel (Solanum tuberosum)-Patatin Gen assoziiert sind, das während der gesamten Kartoffelknollenentwicklung von der Stolonenbildung bis zur reifen Knolle exprimiert wird. Die Transkriptions-Kassette beinhaltet in der 5′-3′ Transkriptionsrichtung eine für die Pflanze repräsentative Region für die Transkription und die Translation, eine beliebige Sequenz und eine Region für die transkriptionale und translationale Termination. Der Terminationsbereich ist beliebig austauschbar.

Die DNA-Sequenz darf alle möglichen offenen Leseraster für ein beliebiges Peptid als auch ein oder mehrere Introns enthalten. Als Beispiel seien genannt: Sequenzen für Enzyme; Sequenzen, die komplementär a) zu einer Genomsequenz sind, wobei die Genomsequenz ein offener Leseraster sein darf; b) zu einem Intron; c) zu einer nicht kodierenden Leitsequenz; d) zu jeder Sequenz, die durch Komplementarität die Transkription, mRNA-Verarbeitungen, (z.B. Splicing) oder die Translation inhibiert. Die gewünschte DNA-Sequenz kann synthetisch hergestellt oder natürlich gewonnen sein oder eine Mischung aus synthetischen und natürlichen DNA-Bestandteilen enthalten. Im allgemeinen wird eine synthetische DNA-Sequenz mit Codons erzeugt, die von Pflanzen bevorzugt werden. Diese von Pflanzen bevorzugten Codons können aus Codons mit der höchsten Proteinhäufigkeit bestimmt werden, die in den meisten interessanten Pflanzenspezies exprimiert werden. Bei der Preparation der Transkriptions-Kassette können die verschiedenen DNA-Fragmente manipuliert werden, um eine DNA-Sequenz zu erhalten, die zweckmäßiger Weise in der korrekten Richtung liest und die mit einem korrekten Leseraster ausgestattet ist. Für die Verbindungen der DNA-Fragmente untereinander können an den Fragmentenden Adapter oder Linker eingesetzt werden. Ferner können Manipulationen, die passende Restriktionsstellen bereitstellen oder die überflüssige DNA oder Restriktionsstellen entfernen, eingesetzt werden. Wo Insertionen, Deletionen oder Substitutionen, wie z.B. Transitionen und Transversionen in Frage kommen, können in vitro Mutagenese, Primerrepair, Restriktion oder Ligation verwendet werden.

Bei geeigneten Manipulationen, wie z.B. Restriktion, "chewing-back" oder Auffüllen von Überhängen für "blunt-ends" können komplementäre Enden der Fragmente für die Zusammenfügung und Ligation zur Verfügung gestellt werden. Zur Durchführung der verschiedenen Stufen ist für eine Vergrößerung der DNA-Menge und für die DNA-Analyse, die der Sicherstellung der erwarteten Erfolge der Eingriffe dient, eine Klonierung erforderlich.

Es ist eine große Anzahl Klonierungsvektoren verfügbar, die ein Replikationssystem in E.coli und einen Marker, der eine Selektion der tranformierten Zellen erlaubt, beinhalten. Die Vektoren beinhalten z.B. pBR 332, pUC-Serien, M13 mp-Serien, pACYC 184 usw. Dementsprechend kann die Sequenz an einer passenden Restriktionsstelle in den Vektor eingefügt werden. Das erhaltene Plasmid wird für die Transformation in E.coli verwendet. Die E.coli Zellen werden in einem geeigneten Nährmedium gezüchtet, anschließend geerntet und lysiert. Das Plasmid wird wiedergewonnen. Als Analysenmethoden werden im allgemeinen eine Sequenzanalyse, eine Restriktionsanalyse, Elektrophoresen und weitere biochemisch-molekularbiologische Methoden durchgeführt. Nach jeder Manipulation kann die verwendete DNA-Sequenz restriktioniert und mit der nächsten DNA-Sequenz verbunden werden. Jede Plasmid-Sequenz kann in den gleichen oder anderen Plasmiden kloniert werden. Je nach Einführungsmethode gewünschter Gene in die Pflanze können weitere DNA-Sequenzen erforderlich sein. Werden z.B. für die Transformation das Ti- oder Ri-Plasmid der Pflanzenzelle verwendet, so muß mindestens die rechte Begrenzung, häufig jedoch die rechte und die linke Begrenzung der Ti- und Ri-Plasmid T-DNA, als Flankenbereich der einzuführenden Gene, verbunden werden. Die Verwendung von T-DNA für die Transformation von Pflanzenzellen ist intensiv untersucht und ausreichend in EP 120 516; Hoekema, In: The Binary Plant Vector System Offset-drukkerij Kanters B.V., Alblasserdam, 1985, Chapter V; Fraley, et al., Crit. Rev. Plant Sci., 4:1-46 und An et al., EMBO J. (1985) 4:277-284 beschrieben worden.

Ist die eingeführte DNA erst einmal im Genom integriert, so ist sie dort auch relativ stabil und springt in der Regel nicht mehr heraus. Sie enthält normalerweise einen Selektionsmarker, der den tranformierten Pflanzenzellen Resistenz gegenüber einem Biozid oder einem Antibiotikum wie Kanamycin. G 418, Bleomycin, Hygromycin oder Chloramphenicol u.a. vermittelt. Der individuell verwendete Marker sollte daher die Selektion transformierter Zellen gegenüber Zellen, denen die eingefügte DNA fehlt, gestatten.

Für die Einführung von DNA in eine pflanzliche Wirtszelle stehen eine Vielzahl von Techniken zur Verfügung. Diese Techniken umfassen die Transformation mit T-DNA unter Verwendung von Agrobacterium tumefaciens oder Agrobacterium rhizogenes als Transformationsmittel, die Fusion, die Injektion oder die Elektroporation sowie weitere Möglichkeiten. Werden für die Transformation Agrobakterien verwendet, muß die einzuführende DNA in spezielle Plasmide kloniert werden und zwar entweder in einen intermediären Vektor oder einen binären Vektor. Die intermediären Vektoren können aufgrund von Sequenzen, die homolog zu Sequenzen in der T-DNA sind, durch homologe Rekombination in das Ti-oder Ri-Plasmid integriert werden. Dieses enthält außerdem die für den Transfer der T-DNA notwendige Vir-Region. Intermediäre Vektoren können sich nicht in Agrobakterien replizieren. Mittels eines Helferplasmids kann der intermediäre Vektor auf Agrobacterium tumefaciens übertragen werden (Konjugation). Binäre Vektoren können sich in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al., Mol. Gen. Genet. (1978), 163:181-187). Das als Wirtszelle dienende Agrobakterium soll ein Plasmid, das die Vir-Region trägt, welche für den Transfer der T-DNA in die Pflanzenzelle notwendig ist, enthalten, wobei zusätzliche T-DNA enthalten sein kann. Das so transformierte Bakterium wird zur Transformation von Pflanzenzellen benutzt. Für den Transfer der DNA in die Pflanzenzelle können Pflanzen-Explanate zweckmäßiger Weise mit Agrobacterium tumefaciens oder Agrobacterium rhizogenes kultiviert werden. Aus dem infizierten Pflanzenmaterial (z.B. Blattstücke, Stengelsegmente, Wurzeln, aber auch Protoplasten oder Suspensions-kultivierte Zellen) können dann in einem geeigneten Medium, welches Antibiotika oder Biozide zur Selektion enthalten kann, wieder ganze Pflanzen regeneriert werden, die dann auf die Anwesenheit der eingeführten DNA getestet werden können. Bei der Injektion und Elektroporation sind keine speziellen Anforderungen an die Plasmide gestellt, es können einfache Plasmide, wie z.B. pUC-Derivate, benutzt werden.

Für die Einführung fremder Gene in die Pflanzen steht eine Vielzahl von Möglichkeiten offen, besonders interessant ist jedoch die Expression von Genen für Säugerprodukte, wie z.B. Blutfaktoren; Lymphokine; Koloniestimulationsfaktoren; Interferone; Plasminogen-Aktivatoren; Enzyme, wie z.B. Superoxid-Dismutase oder Chymosin; Hormone; Thioesterase-2 aus Rattenmilch; oder Human-Serum-Albumin. Eine weitere Möglichkeit ist die Erhöhung der Menge an Knollenproteinen, insbesondere mutierte Knollenproteine, die eine optimierte Aminosäurezusammensetzung (essentielle Aminosäuren) aufweisen, und auf diese Weise den Nährwert der Knollen steigern können.

Soll die Menge bestimmter endogener Produkte verringert werden, ist außerdem die Expression der Gene oder von Teilen dieser Gene in falscher Orientierung zum Promotor denkbar, was zur Synthese einer RNA führt, die komplementar zu einem gesamten oder zu Teilen eines endogenen Gens ist und dadurch die Transkription dieses Gens oder die Prozessierung und/oder Translation der endogenen mRNA hemmen kann.

Die transformierten Zellen wachsen innerhalb der Pflanzen in der üblichen Weise (siehe auch McCormick et al., Plant Cell Reports (1986) 5, 81-84). Diese Pflanzen konnen normal angezogen werden und mit Pflanzen, die die gleiche transformierte Erbanlage oder andere Erbanlagen besitzten, gekreuzt werden. Die daraus entstehenden hybriden Individuen haben die entsprechenden phänotypischen Eigenschaften. Es sollten zwei oder mehrere Generationen angezogen werden, um sicherzustellen, daß das phänntypische Merkmal stabil beibehalten und vererbt wird. Auch sollten Samen geerntet werden, um sicherzustellen, daß der entsprechende Phänotyp oder andere Eigenarten enthalten sind. Als Wirtspflanze für die knollenspezifische Expression sind alle stamm- oder wurzelknollenbildenden Pflanzenspezies geeignet, insbesondere Solanum tuberosum.

Die Identifizierung brauchbarer transkriptionaler Startbereiche kann auf einer Vielzahl von Wegen erreicht werden. Man bedient sich hier in der Regel der mRNAs, die aus bestimmten Teilen der Pflanze (Knolle) isnliert worden sind. Für die zusätzliche Konzentrationssteigerung der spezifisch an das Gewebe oder den pflanzlichen Zustand assoziierten mRNA kann cDNA präpariert werden, wobei unspezifische cDNA an der mRNA oder der cDNA aus anderen Geweben oder Pflanzenzuständen (z.B. Knolle/Blatt) abgezogen werden kann. Die restliche cDNA kann dann für die Sondierung des Genoms der Komplementärsequenzen, unter Benutzung einer geeigneten Pflanzen-DNA-Bibliothek verwendet werden. Wo das Protein isoliert ist oder isoliert wird, kann es partiell sequenziert werden, so daß eine Sonde zur direkten Identifizierung der entsprechenden Sequenzen in einer Pflanzen-DNA-Bibliothek hergestellt werden kann. Anschließend können die Sequenzen, die mit der Sonde hybridisieren, isoliert und manipuliert werden. Ferner kann der nicht translatierte 5′-Bereich, der mit dem kodierenden Bereich assoziiert ist, isoliert und in Expressions-Kassetten für die Identifizierung der transkriptionalen Aktivität des nicht translatierten 5′-Bereichs verwendet werden.

Die gewonnenen Expressions-Kassetten, die die nicht übersetzte 5'-Region verwenden, können in Pflanzen transformiert werden (s.o.), um ihre Funktionsfähigkeit mit einem heterologen Strukturgen (anders als der offene Leseraster des Wildtyps, der mit der nicht übersetzten 5′-Region assoziiert ist) sowie die Knollenspezifität zu prüfen. Auf diese Weise können spezifische Sequenzen, die für die knollenspezifische Transkription notwendig sind, identifiziert werden. Expressions-Kassetten, die von besonderem Interesse sind, enthalten transkriptionale Initiationsstellen der Patatin-Gene.

### Begriffe und Abkürzungen

#### Abkürzungen

| | |
|---|---|
| d,kd = | Dalton, Kilodalton |
| bp = | Basenpaare |
| DNA = | Desoxyribonukleinsäure, Träger der genetischen Information |
| cDNA = | Durch Reverse - Transkriptase hergestellte Kopie einer mRNA |
| mRNA = | Messenger (Boten) - Ribonukleinsäure |
| T-DNA = | Transfer-DNA (auf dem Ti-Plasmid von Agrobacterium tumefaciens lokalisiert) |

#### Begriffe

| | |
|---|---|
| blunt-ends = | DNA-Enden, in denen beide DNA-Stränge exakt gleich lang sind. |
| chewing-back = | enzymatische Entfernung von Nukleotiden eines DNA-Stranges, der länger als der komplementäre Strang eines DNA-Moleküls ist. |
| Elektrophorese = | biochemisches Trennverfahren zur Trennung von Proteinen und Nukleinsäuren nach Größe und Ladung. |
| Expression = | Aktivität eines Gens. |
| Gen = | Erbfaktor; eine Einheit des Erbguts, Träger der Teilinformation für ein bestimmtes Merkmal. Gene bestehen aus Nukleinsäuren (z.B. DNA, RNA). |
| Genom = | Gesamtheit der auf den Chromosomen der Zelle lokalisierten Gene. |
| Genom-Sequenz = | DNA-Sequenz des Genoms, wobei drei hintereinanderliegende Nukleotidbasen (Triplett) ein Codon bilden, welches wiederum für eine bestimmte Aminosäure kodiert. |

EP 0 375 092 B1

| | |
|---|---|
| RNA-Splicing = | ein Gen liegt nicht immer als kolineare Einheit vor, sondern kann nichtkodierende Sequenzen (Introns) enthalten, die aus der mRNA herausgespalten (Splicing) werden müssen. |
| heterologe(s) Gen(e) oder DNA = | Fremd-Gen oder Fremd-DNA |
| homologe(s) Gen(e) oder DNA = | Gen oder DNA stammt aus der gleichen Spezies |
| Klon = | Zellpopulation, die von einer einzigen Mutterzelle stammt. Die Nachkommen sind genotypisch gleich. Durch Klonierung kann die Einheitlichkeit von Zellinien noch gesteigert werden. |
| Ligation = | enzymatische Bildung einer Phosphodiesterbindung zwischen 5′-Phosphatgruppen und 3′-Hydroxylgruppen der DNA. |
| Linker,Polylinker = | synthetische DNA-Sequenz, die eine oder mehrere (Polylinker) Restriktionsschnittstellen in unmittelbarer Reihenfolge enthält. |
| Northern Blots, Southern Blots = | Transfer und Fixierung elektrophoretisch aufgetrennter RNA oder DNA auf eine Nitrozellulose- oder Nylonmembran. |
| Patatin = | Trivialname für das Hauptspeicherprotein der Kartoffelknolle, ein Glycoprotein von ca. 40 kd Molekulargewicht. |
| Phänotyp = | Summe der Merkmale, die in einem Organismus im Gegensatz zu seiner Genaustattung (Genotyp) ausgeprägt ist. |
| Plasmid = | zusätzlicher, extrachromosomaler DNA-Erbträger in Bakterienzellen (evtl. auch in Eukaryonten), der sich unabhängig vom Bakterienchromosom repliziert. Das Plasmid kann in andere Wirts-DNA integriert werden. |
| Primer = | Startstück; Polynukleotidstrang, an dem weitere Nukleotide angehängt werden können. |
| Promotor = | Kontrollsequenz der DNA-Expression, die die Transkription homologer oder heterologer DNA-Gen-Sequenzen gewährleistet. |
| Replikation = | Verdopplung der DNA-Sequenz |
| Restriktionsenzyme = | Restriktionsendonukleasen, die eine Untergruppe der Endozyme desoxyribonukleasen sind (z.B. EcoRI (Spezifität G↓AATTC und EcoRII↓CC($^A_T$) GG, aus E.coli), zeichnen sich durch eine hohe Spezifität der Substraterkennung aus (↓= Spaltstelle). |
| Restriktionsstellen = | Spaltstellen, die spezifisch durch Restriktionsenzyme erstellen zeugt werden. |
| Termination = | letzte Stufe der Proteinsynthese, in der die Polypeptidkette vervollständigt wird. |
| Transformation = | Einfügung exogener DNA eines Bakterienstammes in eine Empfängerzelle. |
| Transkription = | Überschreibung der auf der DNA enthaltenen genetischen Information auf eine RNA. |
| Translation = | Übersetzung der genetischen Information, die in Form einer linearen Abfolge von Basen in Nukleinsäuren gespeichert ist. Das Produkt der Übersetzung ist ein Polypeptid, das aus einer Abfolge von Aminosäuren besteht. |
| Transition = | Basenpaaraustausch: Purin-Pyrimidin zu Purin-Pyrimidin, z.B. A-T durch G-C |
| Transversion = | Basenpaaraustausch: Purin-Pyrimidin zu Pyrimidin-Purin, z.B. A-T durch T-A |
| Deletion = | Entfernung eins oder mehrerer Basenpaare |
| Insertion = | Einbau eins oder mehrerer Basenpaare; Transition, Transversion, Deletion und Insertion sind Punktmutationen. |
| Vektoren = | wirtsspezifische, replizierfähige Strukturen, die Gene aufnehmen und diese auf andere Zellen übertragen. Plasmide können als Vektoren benutzt werden. |

Am 16.12.1988 wurde bei der Deutschen Sammlung von Mikroorganismen (DSM) in Braunschweig, Bundesrepublik Deutschland, folgender Mikroorganismus hinterlegt (Hinterlegungsnummer):

Agrobacterium tumefaciens LBA4404, A-tum B33 enthaltend das Plasmid pBI 101-B33 (DSM 5089).


Beschreibung der Abbildung

Fig. 1    zeigt die Restriktionskarte des genomischen Klons der das Patatin Gen B33 kodiert.

Abkürzungen:

E = Eco RI, H = HindIII, K= KpnI, B = Bam HI, S = SstI,

V = Eco RV, X = Xbal, C = Clal, D = Dral

Fig. 2    zeigt die Nukleinsäuresequenz der für die transkriptionale Regulation wichtigen Bereiche des Patatin-Gens. In der Sequenz ist die Lage des Dral/Dral-Fragments zwischen der Position +14 und der Position -1513 z.B. durch Pfeil eingezeichnet. ATG bezeichnet den Start der Translation (durch ▼ gekennzeichnet).

Fig. 3    zeigt das 13,5 kb lange Plasmid PBI101-B33 mit dem darin enthaltenen 2,0 kb langen Kanamycinresistenz Gen, dem 1,527 kb langen Patatin-Promotor B 33, dem 2,0 kb langen β-Glucuronidase Gen und dem Nopalin-Synthase Terminator.

Zum besseren Verständnis der dieser Erfindung zugrundeliegenden Ausführungsbeispiele wird vorab eine Aufstellung aller für diese Versuche notwendigen und an sich bekannten Verfahren gegeben:

## 1. Klonierungsvektoren

Zur Klonierung wurden die Vektoren pUC18/19 und M13mp18/19 (Yanisch-Perron et al., Gene (1985), 33, 103-119) benutzt.

Für die Pflanzentransformation wurden die Genkonstruktionen in den binären Vektor BIN19 (Bevan, Nucl. Acids. Res. (1984), 12, 8711-8720) kloniert.

## 2. Bakterienstämme

Für die pUC- und M13-Vektoren wurden die E.coli-Stämme BMH71-18 (Messing et al., Proc. Nat. Acad. Sci. USA (1977), 24, 6342-6346) oder TB1 benutzt. Fur den Vektor BIN19 wurde ausschließlich der Stamm TB1 benutzt. TB1 ist ein Rekombinations-negatives, Tetracyclin-resistentes Derivat des Stammes JM101 (Yanisch-Perron et al., Gene (1985), 33, 103-119). Der Genotyp des TB1-Stammes ist (Bart Barrel, pers. Mitteilung): F'(traD36, proAB, lacl, lacZΔM15), Δ(lac, pro), SupE, thiS, recA, Sr1::Tn10(Tc$^R$).

Die Pflanzentransformation wurde mit Hilfe des Agrobacterium tumefaciens-Stammes LBA4404 (Bevan, M., Nucleic Acids Research 12, 8711-8721 (1984); BIN19 Derivat) durchgeführt.

## Medien:

YT-Medium:    0,5 % Hefe-Extrakt, 0,5 % NaCl, 0,8 % Bacto-Trypton, evtl. 1,5 % Agar

YEB-Medium:    0,5 % Rindfleisch-Extrakt, 0,1 % Hefe-Extrakt, 0,5 % Pepton, 0,5 % Saccharose, 2 mM MgSO$_4$, evtl. 1,5 % Agar

MS-Medium:    nach Murashige und Skoog (Physiologia Plantarum (1962), 15, 473 497)

## 3. Transformation von Agrobacterium tumefaciens

Bei Bin19-Derivaten erfolgt die Einführung der DNA in die Agrobakterien durch direkte Transformation nach der Methode von Holsters et al. (Mol. Gen. Genet. (1978), 163, 181-187). Die Plasmid-DNA transformierter Agrobakterien wurde nach der Methode von Birnboim und Doly (Nucl. Acids Res. (1979), 7, 1513-1523) isoliert und nach geeigneter Restriktionsspaltung gelelektrophoretisch aufgetrennt.

## 4. Pflanzentransformation

10 kleine, mit einem Skalpell verwundete Blätter einer Kartoffel-Sterilkultur wurden in 10 ml MS-Medium mit 2 % Saccharose gelegt, welches 30-50 μl einer unter Selektion gewachsenen Agrobacterium tumefaciens-Übernachtkultur enthielt. Nach 3-5 minütigem, leichtem Schütteln wurden die Petrischalen bei 25 °C im Dunkeln inkubiert. Nach 2 Tagen wurden die Blätter auf MS-Medium mit 1,6 % Glukose, 2 mg/l Zeatinribose, 0,02 mg/l Naphthylessigsäure, 0,02 mg/l Giberellinsäure, 500 mg/l Claforan, 50 mg/l Kanamycin und 0,8 % Bacto-Agar ausgelegt. Nach einwöchiger Inkubation bei 25 °C und 3000 Lux wurde die Claforankonzentration im Medium um die Hälfte reduziert.

## 5. Analyse genomischer DNA aus transgenen Pflanzen

Die Isolierung genomischer Pflanzen-DNA erfolgte nach Rogers und Bendich (Plant Mol. Biol. (1985), 5, 69-76).

Für die DNA-Analyse wurden 10-20 μg DNA nach geeigneter Restriktionsspaltung mit Hilfe von "Southern Blots" auf Integration der zu untersuchenden DNA-Sequenzen untersucht.

6. Analyse der Gesamt-RNA aus transgenen Pflanzen

Die Isolierung pflanzlicher Gesamt-RNA erfolgte nach Logemann et al. (Analytical Biochem. (1987), 163, 16-20). Für die Analyse wurden je 50 µg Gesamt-RNA mit Hilfe von "Northern Blots" auf die Anwesenheit der gesuchten Transkripte untersucht.

7. GUS-Test

Die Aktivität der β-Glucuronidase (GUS) in transgenen Pflanzen wurden nach der Methode von Jefferson (Plant Mol. Biol. Rep. (1987), 5, 387-405) bestimmt. Die Proteinbestimmung erfolgte nach der Methode von Bradford (Anal. Biochem. (1976), 72, 248-254). Für die Bestimmung der GUS-Aktivität wurden 50 µg Protein eingesetzt, die Inkubation erfolgte bei 37 °C für 30 Minuten.

Die nachfolgenden Ausführungsbeispiele erläutern die Isolierung und Identifizierung sowie die Funktion und Verwendung des Patatin-Promotors aus Kartoffelknollen.

Ausführungsbeispiel 1

Klonierung und Strukturanalyse eines Patatin-Gens aus Solanum tuberosum

cDNA-Klone, die für das Patatin-Protein der Kartoffel kodieren, wurden aus der Kartoffel-Varietät "Berolina" isoliert und sequenziert (Rosahl et al., Mol. Gen. Genetics 203, 214-220 (1986). Diese cDNA-Klone dienten dazu, einen homologen, genomischen Patatin Klon aus der Kartoffelvarietät Berolina (Max-Planck-Institut für Züchtungsforschung, Köln) zu isolieren.

Ausführungsbeispiel 2

Klonierung, Identifizierung und Primärstruktur eines genomischen Patatinklons

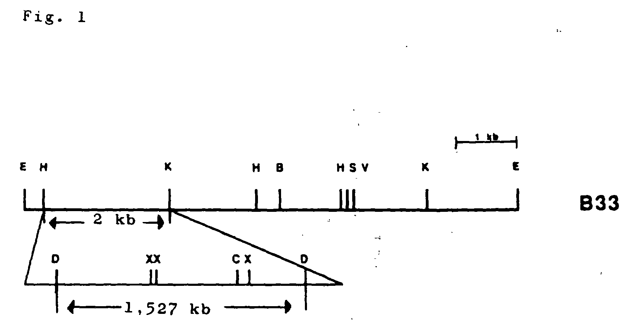

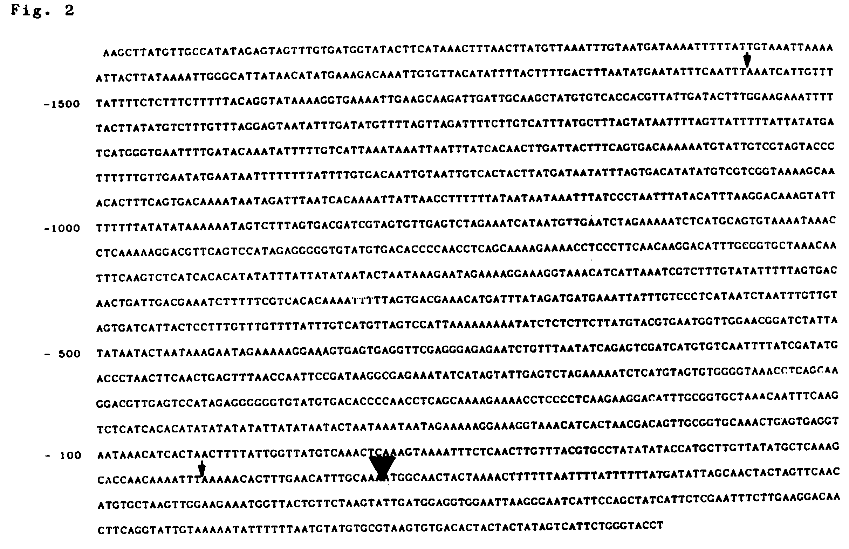

Eine genomische Bibliothek der nuklearen DNA aus der Kartoffelvarietät Berolina, die im vom Lambda-Phagen abgeleiteten Vektor EMBL 4 etabliert worden war, wurde mit Hilfe der Patatin cDNA pcT 58 gescreent. Dreizehn unabhängige Klone wurden erhalten, von denen nach partieller Sequenzierung der Klon B33 für die weiteren Arbeiten verwendet wurde. Die Restriktionskarte des Klons B33 ist in Fig. 1 wiedergegeben. Ein Teil des Gens wurde sequenziert, die Sequenz der für die transkriptionale Regulation wichtigen Bereiche ist in Fig. 2 wiedergegeben.

Ausführungsbeispiel 3

Identifizierung der für die knollenspezifische Expression des Patatin Gens B33 verantwortlichen regulatorischen Bereiche

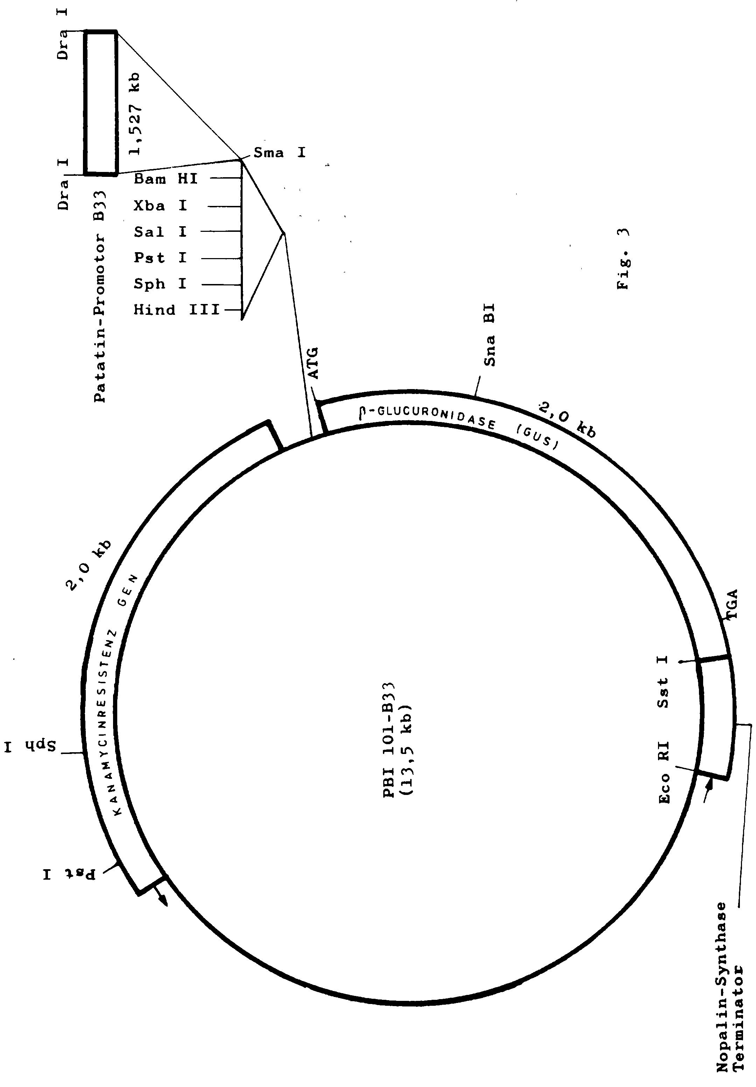

Ein 1,527 kb langes Dral/Dral-Fragment, welches zwischen der Position +14 und Position -1513 lokalisiert ist (s. Fig. 2) wurde in die Smal Schnittstelle des Plasmids pBI101 (Jefferson et al, EMBO J. 6, 3901-3907 (1987)) gesetzt. Dadurch wurde dieses Promotorfragment des Patatin Gens B33 mit dem kodierenden Bereich der β-Glucuronidase aus E.coli und der poly-A enthaltenden Region des Nopalin-Synthase Gens fusioniert (s. Fig. 3). Dieses Konstrukt wurde in den Agrobacterienstamm LBA 4404 (Bevan, M., Nucl. Acids Res. 12, 8711-8721 (1984) transferiert und die das chimäre Patatingen enthaltenden Agrobacterien zur Transformation von Kartoffelblättern eingesetzt.

Von zehn unabhängig erhaltenen Transformanten, in denen die Präsenz des intakten, nicht rearrangierten chimären Patatin-Glucuronidase-Gens mit Hilfe von Southern Blot Analysen nachgewiesen worden war, wurden Blatt, Stamm, Knollen und Wurzel auf die Aktivität der β-Glucuronidase untersucht.

Die Ergebnisse sind in Tabelle 1 wiedergegeben. Aus diesen Daten ergibt sich eindeutig, daß das Dral/Dral-Fragment des Patatin Gens B33, welches mit dem β-Glucuronidase Gen fusioniert worden war, eine starke, knollenspezifische Aktivität der β-Glucuronidase bewirkt.

Tabelle 1

| Glucuronidase Aktivität des chimären B33-Glucuronidase Gens in verschiedenen Organen verschiedener transgener Kartoffelpflanzen | | | | |
|---|---|---|---|---|
| Transformante | Wurzel | Stamm | Blatt | Knolle |
| 33G-13 | 137 | 55 | 0 | 16882 |
| 33G-19 | 138 | 7 | 14 | 2047 |
| 33G-21 | 155 | 1034 | 25 | 19471 |
| 33G-23 | 0 | 50 | 0 | 12149 |
| 33G-24 | 0 | 14 | 0 | 4530 |
| 33G-27 | 86 | 8 | 4 | 7284 |
| 33G-38 | 30 | 14 | 6 | 3847 |
| 33G-52 | 69 | 10 | 0 | 2864 |
| 33G-61 | 31 | 10 | 2 | 14916 |
| 33G-62 | 133 | 151 | 24 | 18620 |
| × | 76 | 135 | 7,5 | 11948 |

c.v. Desiree

Aktivitäten sind in pMol methylumbelliferrol/mg Protein/Minute angegeben.

c.v. Desiree zeigt die entsprechende Aktivät in einer nichttransformierten Kartoffelpflanze.

**Patentansprüche**

1. Verwendung des regulatorischen Bereichs (Promotor) des auf dem KpnI/HindIII-Fragment des Patatingens B33 zwischen der Position +14 und der Position -1513 (Pfeile) liegenden 1,527 kb langen DraI/DraI Fragments mit der folgenden Nukleotidsequenz

```
         AAGCTTATCTTGCCATATAGAGTAGTTTCTGATCCTATACTTCATAAACTTTAACTTATCTTAAATTTGTAATGATAAAATTTTTATTGTAAATTAAAA
                                                                                              ↓
         ATTACTTATAAAATTGCGCATTATAACATATGAAAGACAAATTGTGTTACATATTTTACTTTTCACTTTAATATGAATATTTCAATTTAAATCATTGTTT
  -1500  TATTTTCTCTTTCTTTTTACAGGTATAAAAGCTGAAAATTGAAGCAAGATTCATTGCAAGCTATCTGTCACCACGTTATTCATACTTTGGAAGAAATTTT
         TACTTATATGTCTTTGTTTACGAGTAATATTTGATATGTTTTAGTTAGATTTTCTTGTCATTTATGCTTTAGTATAATTTTAGTTATTTTTATTATATGA
         TCATCGGTGAATTTTCGATACAAATATTTTTGTCATTAAATAAATTAATTTATCACAACTTGATTACTTTCAGTGACAAAAAATGTATTGTCGTAGTACCG
         TTTTTTCTTCAATATGAATAATTTTTTTTTATTTTGTGACAATTGTAATTGTCACTACTTATGATAATATTTAGTGACATATATGTCGTCGGTAAAAGCAA
         ACACTTTCACTCACAAAATAATAGATTTAATCACAAAATTATTAACCTTTTTTATAATAATAAATTTATCCCTAATTTATACATTTAAGCACAAAGTATT
  -1000  TTTTTTATATATAAAAAATAGTCTTTAGTGACCATCGTAGTCTTCAGTCTACAAATCATAATGTTGAATCTAGAAAAATCTCATCCAGTCTAAAATAAAC
         CTCAAAAAGGACCTTCAGTCCATAGAGGGGCGTGTATGTGACACCCCAACCTCAGCAAAAGAAAACCTCCCTTCAACAAGGACATTTGCGGTGCTAAACAA
         TTTCAAGTCTCATCACACATATATTTATTATATAATACTAATAAAGAATAGAAAAGGAAAGGTAAACATCATTAAATCGTCTTTGTATATTTTTAGTCAC
         AACTGATTGACGAAATCTTTTTCCTCACACAAAATTTTTAGTGACGAAACATGATTTATAGATGATGAAATTATTTGTCCCTCATAATCTAATTTGTTGT
         AGTGATCATTACTCCTTTGTTTGTTTTATTTGTCATGTTAGTCCATTAAAAAAAAAATATCTCTCTTCTTATGTACGTGAATGGTTGGAACGGATCTATTA
  - 500  TATAATACTAATAAAGAATAGAAAAAGGAAAGTGACTGACGTTCGAGGGACAGAATCTGTTTAATATCAGAGTCGATCATGTGTCAATTTTATCGATATG
         ACCCTAACTTCAACTGACTTTAACCAATTCCGATAAGGCGAGAAATATCATAGTATTGAGTCTAGAAAAATCTCATCGTAGTCTGGGGTAAACGTCAGGAA
         GGACGTTCAGTCCATAGAGCGGCGCTGTATCTGACACCCCAACCTCAGCAAAAGAAAACCTCCCCTCAAGAAGGACATTTGCGGTGCTAAACAATTTCAAG
         TCTCATCACACATATATATATATTATATAATACTAATAAATAATAGAAAAAGGAAAGGTAAACATCACTAACGACAGTTGCCGTGCAAACTCAGTGAGGT
  - 100  AATAAACATCACTAACTTTTATTGGTTATCTCAAACTCAAAGTAAAATTTCTCAACTTGTTTACGTGCCTATATATACCATGCTTGTTATATGCTCAAAG
                ↓
         CACCAACAAAATTTAAAAAACACTTTGAACATTTGCAAAATGGCAACTACTAAAACTTTTTTAATTTTATTTTTTATGATATTAGCAACTACTAGTTCAAC
         ATGTCCTAACTTGGAAGAAATGGTTACTGTTCTAAGTATTGATCGACGTGGAATTAAGGGAATCATTCCAGCTATCATTCTCGAATTTCTTGAAGGACAA
         CTTCAGGTATTGTAAAAATATTTTTTAATGTATGTCGCCGTAAGTGTGACACTACTACTATAGTGATTGTGGGTACCT
```

zur Transformation von Kulturpflanzen.

2. Verwendung der Patatingen Promotor-Sequenz gemäß Anspruch 1, zur Regulierung endogener und zur Herstellung heterologer Produkte in Kulturpflanzen.

3. Verwendung der Patatingen Promotor-Sequenz gemäß Anspruch 1, zur knollenspezifischen Expression in Kulturpflanzen.

4. Verwendung der Patatingen Promotor-Sequenz gemäß den Ansprüchen 1 - 3, dadurch gekennzeichnet, daß die Kulturpflanze eine Kartoffel ist.

5. <u>Agrobacterium</u> <u>tumefaciens</u> LBA 4404 A-tum B33 (DSM 5089).

6. Eine transgene Kartoffel, die sich von einer nicht transformierten Kartoffel durch eine erhöhte Expression endogener und heterogener Produkte in der Knolle unterscheidet, dadurch gekennzeichnet, daß in der transgenen Pflanze hinter dem regulatorischen Bereich (Promotor) des auf dem KpnI/HindIII-Fragments des Patatingens B33 zwischen der Position +14 und der Position -1513 liegenden 1,527 kb langen DraI/DraI Fragments der Sequenz gemäß Anspruch 1, eine Sequenz nachgeschaltet ist, die den Phänotyp der betreffenden Zellgewebe modifiziert und die Information zur Bildung endogener und heterogener Expressionsprodukte für eine neue Funktion enthält, wobei in Transkriptionsrichtung ferner ein Linker mit mindestens 5bp, der ein oder mehrere Restriktionsstellen für die Insertion der nachgeschalteten Sequenz aufweist, enthalten ist.

**Claims**

1. Use of the regulatory region (promoter) of the 1,527 kb long DraI/DraI fragment which is located on the KpnI/HindIII-fragment of the patatin gene B33 between the position +14 and the position -1513 (arrows), having the following nucleotide sequence

```
      AAGCTTATGTTGCCATATAGAGTAGTTTGTGATGGTATACTTCATAAACTTTAACTTATGTTAAATTTGTAATGATAAAATTTTTATTGTAAATTAAAA
      ATTACTTATAAAATTGGGCATTATAACATATGAAAGACAAATTGTGTTACATATTTTACTTTTGACTTTAATATGAATATTTCAATTTAAATCATTGTTT
-1500 TATTTTCTCTTTCTTTTTACAGGTATAAAAGGTGAAAATTGAAGCAAGATTGATTGCAAGCTATGTGTCACCACGTTATTGATACTTTGGAAGAAATTTT
      TACTTATATGTCTTTGTTTAGGAGTAATATTTGATATGTTTTAGTTAGATTTTCTTGTCATTTATGCTTTAGTATAATTTTAGTTATTTTTATTATATGA
      TCATGGGTGAATTTTGATACAAATATTTTTGTCATTAAATAAATTAATTTATCACAACTTGATTACTTTCAGTGACAAAAAATGTATTGTCGTAGTACCC
      TTTTTTGTTGAATATGAATAATTTTTTTTTATTTTGTGACAATTGTAATTGTCACTACTTATGATAATATTTAGTGACATATATGTCGTCGGTAAAAGCAA
      ACACTTTCAGTCACAAAATAATAGATTTAATCACAAAATTATTAACCTTTTTTATAATAATAAATTTATCCCTAATTTATACATTTAAGGACAAAGTATT
-1000 TTTTTTATATATAAAAAATAGTCTTTAGTGACGATCGTAGTGTTGAGTCTAGAAATCATAATGTTGAATCTAGAAAAATCTCATGCAGTCTAAAATAAAC
      CTCAAAAAGGACGTTCAGTCCATAGAGGGGGTGTATGTGACACCCCAACCTCAGCAAAAGAAAACCTCCCTTCAACAAGGACATTTGCGGTGCTAAACAA
      TTTCAAGTCTCATCACACATATATTTATTATATAATACTAATAAAGAATAGAAAAGGCAAAGGTAAACATCATTAAATCGTCTTTGTATATTTTTAGTGAC
      AACTGATTGACGAAATCTTTTTCGTCACACAAAATTTTTAGTGACGAAACATGATTTATAGATGATGAAATTATTTGTCCCTCATAATCTAATTTGTTGT
      AGTGATCATTACTCCTTTGTTTGTTTTATTTGTCATGTTAGTCCATTAAAAAAAAAATATCTCTCTTCTTATGTACGTGAATGGTTGGAACGGATCTATTA
- 500 TATAATACTAATAAAGAATAGAAAAGGCAAAGTGAGTGAGGTTCGAGGGCAGAGAATCTGTTTAATATCAGAGTCGATCATGTGTCAATTTTATCGATATG
      ACCCTAACTTCAACTGACTTTAACCAATTCCGATAAGCGCAGAAATATCATAGTATTGAGTCTAGAAAAATCTCATGTAGTGTGGGGTAAACCTCAGCAA
      GGACGTTCAGTCCATAGAGGGGGGTGTATGTGACACCCCAACCTCAGCAAAAGAAAACCTCCCCTCAAGAAGGACATTTGCGGTGCTAAACAATTTCAAG
      TCTCATCACACATATATATATATTATATAATACTAATAAATAATAGAAAAAGGAAAGGTAAACATCACTAACGACAGTTGCGGTGCAAACTGAGTGACGT
- 100 AATAAACATCACTAACTTTTATTGGTTATGTCAAACTCAAAGTAAAATTTCTCAACTTGTTTACGTGCCTATATATACCATGCTTGTTATATGCTCAAAG
      CACCAACAAAATTTAAAAACACTTTGAACATTTGCAAAATGGCAACTACTAAAACTTTTTTAATTTTATTTTTTATGATATTAGCAACTACTAGTTCAAC
      ATGTGCTAAGTTGGAAGAAATGCTTACTGTTCTAAGTATTGATGGAGGTGGAATTAAGGGAATCATTCCAGCTATCATTCTCGAATTTCTTGAAGGACAA
      CTTCAGGTATTGTAAAAAATATTTTTTAATGTATGTGCCGTAAGTGTGACACTACTACTATAGTCATTCTGGGTACCT
```

for the transformation of cultivated plants.

2. Use of the patatin gene promoter sequence, according to claim 1, for the regulation of endogenous and the production of heterologous products in cultivated plants.

3. Use of the patatin gene promoter sequence, according to claim 1, for tuber specific expression in cultivated plants.

4. Use of the patatin gene promoter sequence, according to claims 1 to 3, characterised in that the cultivated plant is a potato.

5. Agrobacterium tumefaciens LBA 4404 A-tum B33 (DSM 5089).

6. A transgenic potato which differs from an untransformed potato with respect to a higher expression of endogenous and heterologous products in the tuber, characterised in that in the transgenic plant behind the regulatory region (promoter) of the 1,527 kb long Dral/Dral fragment, which is located on the Kpnl/HindIII fragment of the patatin gene B33 between the position +14 and -1513, having the sequence according to claim 1, a sequence is connected which modifies the phenotype of the concerning cell tissues and which contains the information for the production of endogenous and heterologous expression products for a new function, whereby in transcriptional direction, further a linker is contained having at least 5 bp, which has one or more restriction positions for the insertion of the connected sequence.

## Revendications

1. Utilisation du domaine de régulation (promoteur) du fragment Dral/Dral de longueur de 1,527 kb situé sur le fragment Kpnl/HindIII du gène Patatin B33 entre la position +14 et la position -1513 (flèche) ayant la séquence de nucléotides suivante

```
            AAGCTTATGTTCCCATATAGAGTACTTTCTGATCGTATACTTCATAAACTTTAACTTATGTTAAATTTGTAATCATAAAATTTTTATTGTAAATTAAAA
            ATTACTTATAAAATTGGGCATTATAACATATCAAAGACAAATTGTCTTACATATTTTACTTTTGACTTTAATATGAATATTTCAATTTAAATCATTGTTT
    -1500   TATTTTCTCTTTCTTTTTACAGGTATAAAAGGTGAAAATTGAAGCAACATTGATTGCAACGTATGTGTCACCACGTTATTGATACTTTGGAACAAATTTT
            TACTTATATGTCTTTGTTTAGGAGTAATATTTGATATGTTTTACTTACATTTTCTTGTCATTTATGCTTTAGTATAATTTTACTTATTTTTATTATATGA
            TCATGGGTGAATTTTCATACAAATATTTTTTGTCATTAAATAAATTAATTTATCACAACTTCATTACTTTCAGTGACAAAAAATGTATTCTGGTAGTAGGG
            TTTTTTGTTCAATATGAATAATTTTTTTTATTTTGTGACAATTGTAATTGTCACTACTTATGATAATATTTAGTGACATATATGTGGTGGGTAAAAGGAA
            ACACTTTCAGTGACAAAAATAATAGATTTAATCACAAAATTATTAACCTTTTTTTATAATAATAAATTTATCCCTAATTTATACATTTAAGGACAAAGTATT
    -1000   TTTTTTATATATAAAAAATAGTGTCTTTAGTGACGATCGTAGTGTTGACTCTAGAAATCATAATGTTGAATGTAGAAAAATGTCATGGAGTGTAAAATAAAG
            CTCAAAAAGGACGTTGAGTGCATAGAGGCGGGTGTATGTGACACCCGAAGCTGAGGCAAAAGAAAACGTGCGCTTGAAGAAGGGACATTTGGGGTGGTAAAGAA
            TTTCAAGTGTGATGACAGAGATATATTTATTTATATATAATAGTAATAAAGGAATAGAAAAGGAAGGTAAAGATGATTAAATGGTGTTGTTCTATATTTTTTAGTGAG
    30      AACTGATTGAGGAAATGTTTTTGGGGTAGAGACAAAAATTTTTAGTGAGCGAAAGATGGATTTATAGATGATGAAATTATTTGTGGGGTGATAATGTAATTTTGTTGT
            AGTGATGATTAGTGGTTTTGTTGTTTTATTTGTGATGTTAGTGGATTAAAAAAAAAAAATATGTGTGTTGTTATGTAGGTGAATGGTTGGAAGGGATGTATTA
    - 500   TATAATAGTAATAAAGAATAGAAAAAGGAAAGTGAGTGAGGTTGGAGGGAGAGAATGTGTGTTTAATATGAGAGTGGATGATGTGTGTGAATTTTTATGGATATG
            AGGGTAAGTTGAAGTGAGTTTTAAGGAATTGGGATAAGGGGAGAAAATATGATAGTATTGAGTGTAGAAAAATGTGATGTAGTGTGGGGGTAAAGGTGAGGAA
            GGAGGTTGAGTGGATAGAGGGGGGTGTATGTGAGAGAGGGGGAAGGTGAGGGAAAAGAAAAGGTGGGGGTGAAGAAGGGAGATTTGGGGTGGTAAAGAATTTGAAG
            TGTGATGAGAGAGATATATATATATATTATATAATAGTAATAAATAAATAATAGAAAAAGGAAAGGTAAAGATGATGAGTAAGGAGAGTGAGGTTGGGGTGGAAAGTGAGTGAGGT
    - 100   AATAAAGATGAGTAAGTTTTATTGGTTATGTGAAAGTGAAAGTAAAATTTGTGAACTTGTTTAGGTGGGTATATATAGGATGGTTGTTATATGGTGAAAG
            GAGGAAGAAAATTTAAAAAGACTTTGAAGATTTGGGAAAATGGGAAGTAGTAAAAGTTTTTTAATTTTATTTTTTATGATATTAGGAAGTAGTAGTTGAAG
            ATGTGGTAAGTTGGAAGAAAATGGTTAGTGTTGTAAGTATTGATGGAGGTGGAATTAAGGGAATGATTGGAGGTATGATTGTGGAATTTGTTGAAGGAGAA
            GTTGAGGTATTGTAAAAATATTTTTTTAATGTATGTGGGGTAAGTGTGAGAGTAGTAGTATAGTGATTGTGGGTAGGT
```

en vue de la transformation de plantes cultivées.

2. Utilisation de la séquence promoteur du gène Patatin selon la revendication 1, en vue de la régulation de produits endogènes et en vue de la préparation de produits hétérologues dans des plantes cultivées.

3. Utilisation de la séquence promoteur du gène Patatin selon la revendication 1, en vue de l'expression spécifique dans les tubercules de plantes cultivées.

4. Utilisation de la séquence promoteur du gène Patatin selon les revendications 1-3, caractérisé en ce que la plante cultivée est une pomme de terre.

5. Agrobacterium tumefaciens LBA 4404 A-tum B33 (DSM 5089).

6. Pomme de terre transgènique, qui se différencie d'une pomme de terre non transformée par une expression accrue de produits endogènes et hétérogènes dans les tubercules, caractérisé en ce que dans la plante transgènique une séquence est insérée derrière le domaine régulateur (promoteur) du fragment Dral/Dral de la séquence selon la revendication 1, de longueur de 1,527 kb située sur le fragment Kpnl/HindIII du gène Patatin B33 entre la position

EP 0 375 092 B1

+14 et la position -1513, séquence insérée qui modifie le phénotype du tissu cellulaire concerné et qui contient l'information pour la formation de produits d'expression endogènes et hétérogènes pour une nouvelle fonction, une liaison ("Linker") ayant au moins 5bp étant comprise de plus dans la direction de la transcription, ladite liaison possédant une ou plusieurs positions de restriction pour l'insertion de la séquence introduite.

Fig. 1

Fig. 2

AAGCTTATGTTGCCATATAGAGTAGTTTGTGATGGTATACTTCATAAACTTTAACTTATGTTAAATTTGTAATGATAAAATTTTTATTGTAAATTAAAA

ATTACTTATAAAATTGGGCATTATAACATATGAAAGACAAATTGTGTTACATATTTTACTTTTGACTTTAATATGAATATTTCAATTTAAATCATTGTTT

-1500 TATTTTCTCTTTCTTTTTACAGGTATAAAAGGTGAAAATTGAAGCAAGATTGATTGCAAGCTATGTGTCACCACGTTATTGATACTTTGGAAGAAATTTT

TACTTATATGTCTTTGTTTAGGAGTAATATTTGATATGTTTTAGTTAGATTTTCTTGTCATTTATGCTTTAGTATAATTTTAGTTATTTTTATTATATGA

TCATGGGTGAATTTTGATACAAATATTTTTGTCATTAAATAAATTAATTTATCACAACTTGATTACTTTCAGTGACAAAAAATGTATTGTCGTAGTACCC

TTTTTTGTTGAATATGAATAATTTTTTTTTATTTTGTGACAATTGTAATTGTCACTACTTATGATAATATTTAGTGACATATATGTCGTCGGTAAAAGCAA

ACACTTTCAGTGACAAAATAATAGATTTAATCACAAAATTATTAACCTTTTTTATAATAATAAATTTATCCCTAATTTATACATTTAAGGACAAAGTATT

-1000 TTTTTTATATATAAAAAATAGTCTTTAGTGACGATCGTAGTGTTGAGTCTAGAAATCATAATGTTGAATCTAGAAAAATCTCATGCAGTGTAAAATAAAC

CTCAAAAAGGACGTTCAGTCCATAGAGGGGGTGTATGTGACACCCCAACCTCAGCAAAAGAAAACCTCCCTTCAACAAGGACATTTGCGGTGCTAAACAA

TTTCAAGTCTCATCACACATATATTTATTATATAATACTAATAAAGAATAGAAAAGGAAAGGTAAACATCATTAAATCGTCTTTGTATATTTTTAGTGAC

AACTGATTGACGAAATCTTTTTCGTCACACAAAATTTTTAGTGACGAAACATGATTTATAGATGATGAAATTATTTGTCCCTCATAATCTAATTTGTTGT

AGTGATCATTACTCCTTTGTTTGTTTTATTTGTCATGTTAGTCCATTAAAAAAAAAATATCTCTCTTCTTATGTACGTGAATGGTTGGAACGGATCTATTA

- 500 TATAATACTAATAAAGAATAGAAAAAGGAAAGTGAGTGAGGTTCGAGGGAGAGAATCTGTTTAATATCAGAGTCGATCATGTGTCAATTTTATCGATATG

ACCCTAACTTCAACTGAGTTTAACCAATTCCGATAAGGCGAGAAATATCATAGTATTGAGTCTAGAAAAATCTCATGTAGTGTGGGGTAAACCTCAGCAA

GGACGTTGAGTCCATAGAGGGGGGGTGTATGTGACACCCCAACCTCAGCAAAAGAAAACCTCCCCTCAAGAAGGACATTTGCGGTGCTAAACAATTTCAAG

TCTCATCACACATATATATATATTATATAATACTAATAAATAATAGAAAAAGGAAAGGTAAACATCACTAACGACAGTTGCGGTGCAAACTGAGTGAGGT

- 100 AATAAACATCACTAACTTTTATTGGTTATGTCAAACTCAAAGTAAAATTTCTCAACTTGTTTACGTGCCTATATATACCATGCTTGTTATATGCTCAAAG

CACCAACAAAATTTAAAAACACTTTGAACATTTGCAAATGGCAACTACTAAAACTTTTTTAATTTTATTTTTTATGATATTAGCAACTACTAGTTCAAC

ATGTGCTAAGTTGGAAGAAATGGTTACTGTTCTAAGTATTGATGGAGGTGGAATTAAGGGAATCATTCCAGCTATCATTCTCGAATTTCTTGAAGGACAA

CTTCAGGTATTGTAAAAATATTTTTTTAATGTATGTGCGTAAGTGTGACACTACTACTATAGTCATTCTGGGTACCT

Fig. 3